# EUROPEAN PATENT SPECIFICATION

(11) **EP 1 387 667 B1**
(45) Date of publication and mention of the grant of the patent: **26.10.2005**
(21) Application number: 02740558.8
(22) Date of filing: 02.05.2002
(51) Int. Cl.: A61K 7/50, A47K 7/03, C11D 17/04

(54) **DAMP CLEANSING WIPE**
FEUCHTES REINIGUNGSTUCH
LINGETTE DE NETTOYAGE HUMIDE

(30) Priority: 14.05.2001 US 290791 P
(43) Date of publication of application: 11.02.2004
(73) Proprietor: UNILEVER PLC, London EC4P 4BQ (GB); UNILEVER N.V., 3013 AL Rotterdam (NL)
(72) Inventor: LEE, Jae, Trumbull, CT 06611 (US); GOTT, R. E., Unilever Home & Personal Care USA, Trumbull, CT 06611 (US); SLAVTCHEFF, C. S., Unilever Home & Per. Care USA, Trumbull, CT 06611 (US); CHENEY, M. C., Unilever Home & Personal Care USA, Trumbull, CT 06611 (US)
(74) Representative: Mulder, Cornelis Willem Reinier
(86) International application number: PCT/EP2002/004872
(87) International publication number: WO 2002/092050

(56) References cited:
- EP-A- 0 870 496
- WO-A-00/16740
- US-A- 5 951 991

## Description

The invention concerns low-cost, easily manufacturable disposable single use, substantially damp, cleansing articles.

Personal cleansing and conditioning products have traditionally been marketed in a variety of forms such as bar soaps, creams, lotions, and gels. These formulations have attempted to satisfy a number of criteria to be acceptable to consumers. These criteria include cleansing effectiveness, skin feel, skin mildness and lather volume. Ideal personal cleansers should gently cleanse the skin or hair, cause little or no irritation, and not leave the skin or hair overly dry after frequent use.

A series of granted and pending patent applications have been published by Procter & Gamble describing disposable personal cleansing products purportedly addressing many of the aforementioned functionality concerns. These products are substantially dry articles having deposited onto a woven or non-woven cloth a cleansing composition of surfactant, structurant, skin conditioning agent and other performance ingredients. The term "substantially dry" is defined in most of these documents as maximum 10%, but in some instances as high as 15% water. Particularly preferred levels are 5% or less. A commercial embodiment sold in the U.S. is Olay® Daily Facial Cleansing Cloths having water levels of 3-4% by weight of the total cloth article. The technology is described in the following patents.

U.S. Patent 5,951,991 (Wagner et al.) focuses on providing the substrate with a conditioning emulsion separately impregnated from the lathering surfactant onto the cloth substrate. U.S. Patent 5,980,931 (Fowler et al.) emphasizes impregnation of oil soluble conditioning agents. WO 99/55303 (Albacarys et al.) describes skin care actives formulated with the cleansing composition.

Manufacturing processes for these products are reported in U.S. Patent 5,952,043 and U.S. Patent 5,863,663, both to Mackey et al. These patents teach use of a continuous lipid phase with a high melting waxy material deposited onto the wipe substrate. The material is intended to be sufficiently brittle so as to be easily disrupted by low shear contact (e.g. during wiping of the skin) to readily release an internal skin conditioning phase, yet the material is required to be sufficiently tough to avoid premature release of the internal phase during the rigors of processing. A problem with this technology is that through compromise the continuous external lipid phase/internal polar phase is neither sufficiently robust for processing and handling nor sufficiently releasable under wash conditions to allow efficient release of conditioning agent onto the skin.

More recent publications in this area include WO 01/08542 A1 (Cen et al.), WO 01/08655 A1 (Phipps et al.), WO 01/08656 A1 (Smith et al.), WO 01/08657 A1 (Lorenzi et al.) and WO 01/08658 A1 (Cawkwell et al.), all to Procter and Gamble. These documents extend the wipe technology to bonded double layer substrates of contrasting textural properties. A rougher of the two sides may act as a gripping surface while the other may be used for delivering cleansing aids. The articles are described as being substantially dry defined as a Moisture Retention ratio of less than 0.95 gms. The ratio reports weight for total non-bound liquids in the article but is not synonymous with water content. Water levels are not defined.

EP-A-870496 discloses a skin cleansing wipe impregnated with a composition comprising 10% by weight of surfactant 10% by weight of water binding agent (glycerol) and 79 % by weight of water.

Our evaluations of dry wipes produced by the known technology has indicated slow latherability. We attribute the problem to the relatively low water content. A need exists for a cleansing wipe of improved foamability and one which can be efficiently manufactured.

Accordingly, it is an advantage of the present invention to provide a disposable cleansing product which upon contact with water rapidly lathers and generates a rich long lasting foam.

Another advantage of the present invention is to provide a disposable cleansing product having a cleansing composition coatable onto a flexible wiping cloth in a process that minimizes foam generation during manufacture.

Still another advantage of the present invention is to provide a disposable cleansing product which may include an impregnated composition allowing for improved manufacturability, better aesthetics and increased latherability.

These and other advantages of the present invention will become more apparent in light of the following summary and disclosure.

A damp cleansing product is provided which includes:
(i) a water insoluble substrate; and
(ii) a cleansing composition impregnated onto the substrate including:
   (a) at least one surfactant present in an amount from 0.5 to 60% by weight of impregnated composition;
   (b) water; and
wherein the water is present at greater than 15% to 35% by weight of the product, from 0.1 to 60% of a water binding agent.

Now it has been found that improved lather quality/stability, foam height and imparted skinfeel is achieved through the presence of a water-binding agent in such amounts which would lower water activity to a range from less than 0.977, preferably less than about 0.96, more preferably less than about 0.94 and optimally less than about 0.90. Water activity should be at least 0.001, preferably at least about 0.50, and optimally greater than about 0.80.

Illustrative water-binding agents are polyols and inorganic salts. The polyols include glycols, polyglycols, saccharides and polysaccharides. Typical polyols include glycerol (also known as glycerin), polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof.

For best results the water-soluble binding agent is preferably glycerin. Also particularly preferred are polyethylene glycol (average molecular weight ranging from about 200 to about 2,000,000, with PEG-9M and PEG-14M being preferred) and hexylene glycol. In certain types of compositions the latter binding agent may be inappropriate and therefore polyols other than hexylene glycol should be utilized.

Saccharides useful as binding agents for purposes of this invention include dextrose, sucrose, mannose, lactose and fructose and functionalized derivatives thereof including key C₁-C₄₀ alkyl esters, alkyl ethers, sulphates, carboxylates and phosphate derivatives.

Among the suitable inorganic salts are the alkali metal, ammonium, alkaline earth and other metal salts of halides, phosphates, sulphates and any combinations thereof. Particularly preferred is sodium chloride as an inorganic binding agent.

The amount of water-soluble binding agent preferably ranges from about 1 and about 30% by weight of the composition.

Water activity is defined as the vapor pressure of water in a system relative to the vapor pressure of pure water at the same temperature. It is also the equilibrium relative humidity of the air surrounding the system at the same temperature. A product with no "free" water will have a water activity of 0.000. A product such as a pure water coated towelette will have a water activity of 1.000. An instrument for direct measurement of water activity is sold under the name of AquaLab by Decagon Devices Inc., of Pullman, Washington.

The cleansing products of the present invention may have a water content ranging from greater than 15% to about 35%, preferably from about 20% to about 35%, and optimally from about 25% to about 30% by weight of the cleansing product.

Further, the impregnated compositions used in the present invention will have a viscosity ranging from about 0.7g/cm.s (70) to about 3.000 g/cm.s (300,000 cp (centipoise)). Thickness is measured on a Haake CV 20 Rheometer with 30 mm profiled parallel plates at 23°C. A preferred viscosity range is from about 1 g/cm.s (100) to about 2,500 g/cm.s (250,000 cp), more preferably from about 1.5 g/cm.s (150) to about 1,000 g/cm.s (100,000), even more preferably from about 2g/cm.s (200) to about 600g/cm.s (50,000 cp), and optimally from about 4 g/cm.s (400) to about 100 g/cm.s (1,000 cp).

An essential element of compositions according to the present invention is a surfactant. Preferably, these surfactants should be mild, which means that they must provide sufficient cleansing or detersive benefits but not overly dry the skin or hair, and yet meet the lathering criteria described above.

The products of the present invention typically include at least one surfactant in an amount from about 0.5% to about 60%, preferably from about 0.75% to about 40%, and more preferably from about 1% to about 20%, based on the weight of the impregnated composition.

A wide variety of surfactants are useful herein and include those selected from the group consisting of anionic, nonionic, cationic, amphoteric and lathering surfactant mixtures thereof.

Among the lathering surfactants useful herein are the following non-limiting examples which include the classes of:
(1) Alkyl benzene sulfonates in which the alkyl group contains from 9 to 15 carbon atoms, preferably 11 to 14 carbon atoms in straight chain or branched chain configuration. Especially preferred is a linear alkyl benzene sulfonate containing about 12 carbon atoms in the alkyl chain.
(2) Alkyl sulfates obtained by sulfating an alcohol having 8 to 22 carbon atoms, preferably 12 to 16 carbon atoms. The alkyl sulfates have the formula ROSO₃₋M⁺ where R is the C₈₋₂₂ alkyl group and M is a mono- and/or divalent cation.
(3) Paraffin sulfonates having 8 to 22 carbon atoms, preferably 12 to 16 carbon atoms, in the alkyl moiety. These surfactants are commercially available as Hostapur SAS from Hoechst Celanese.
(4) Olefin sulfonates having 8 to 22 carbon atoms, preferably 12 to 16 carbon atoms. Most preferred is sodium C₁₄-C₁₆ olefin sulfonate, available as Bioterge AS 40®
(5) Alkyl ether sulfates derived from an alcohol having 8 to 22 carbon atoms, preferably 12 to 16 carbon atoms, ethoxylated with less than 30, preferably less than 12, moles of ethylene oxide. Most preferred is sodium lauryl ether sulfate formed from 2 moles average ethoxylation, commercially available as Standapol ES-2®.
(6) Alkyl glyceryl ether sulfonates having 8 to 22 carbon atoms, preferably 12 to 16 carbon atoms, in the alkyl moiety.
(7) Fatty acid ester sulfonates of the formula: R¹CH(SO₃₋M+)CO₂R² where R¹ is straight or branched alkyl from about C₈- to C₁₈, preferably C₁₂ to C₁₆, and R² is straight or branched alkyl from about C₁ to C₆, preferably primarily C₁, and M+ represents a mono- or divalent cation.
(8) Secondary alcohol sulfates having 6 to 18, preferably 8 to 16 carbon atoms.
(9) Fatty acyl isethionates having from 10 to 22 carbon atoms, with sodium cocoyl isethionate being preferred.
(10) Dialkyl sulfosuccinates wherein the alkyl groups range from 3 to 20 carbon atoms each.
(11) Alkanoyl sarcosinates corresponding to the formula RCON(CH₃)CH₂CH₂CO₂M wherein R is alkyl or alkenyl of about 10 to about 20 carbon atoms and M is a water-soluble cation such as ammonium, sodium, potassium and trialkanolammonium. Most preferred is sodium lauroyl sarcosinate.
(12) Alkyl lactylates wherein the alkyl groups range from 8 to 12 carbon atoms, with sodium lauroyl lactylate sold as Pationic 138C® available from the Patterson Chemical Company as the most preferred.
(13) Taurates having from 8 to 16 carbon atoms, with cocoyl methyl taurate being preferred.

Nonionic surfactants suitable for the present invention include C₁₀-C₂₀ fatty alcohol or acid hydrophobes condensed with from 2 to 100 moles of ethylene oxide or propylene oxide per mole of hydrophobe; C₂-C₁₀ alkyl phenols condensed with from 2 to 20 moles of alkylene oxides; mono- and di- fatty acid esters of ethylene glycol such as ethylene glycol distearate; fatty acid monoglycerides; sorbitan mono- and di- C₈-C₂₀ fatty acids; and polyoxyethylene sorbitan available as Polysorbate 80 and Tween 80® as well as combinations of any of the above surfactants.

Other useful nonionic surfactants include alkyl polyglucosides, saccharide fatty amides (e.g. methyl gluconamides) as well as long chain tertiary amine oxides. Examples of the latter category are: dimethylododecylamine oxide, oleyldi(2-hydroxyethyl)amine oxide, dimethyloctylamine oxide, dimethyldecylamine oxide, dimethyltetradecylamine oxide, di(2-hydroxyethyl)tetradecylamine oxide, 3-didodecyloxy-2-hydroxypropyldi(3-hydroxypropyl)amine oxide, and dimethylhexadecylamine oxide.

Amphoteric surfactants useful for the present invention include aliphatic secondary and tertiary amines, preferably wherein the nitrogen is in a cationic state, in which the aliphatic radicals can be straight or branched chain and wherein one of the radicals contains an ionizable water solubilizing group such as carboxy, sulphonate, sulphate, phosphate or phosphonate. Illustrative substances are cocamidopropyl betaine, cocamphoacetate, cocamphodiacetate, cocamphopropionate, cocamphodipropionate, cocamidopropyl hydroxysultaine, cetyl dimethyl betaine, cocamidopropyl PG-dimonium chloride phosphate, coco dimethyl carboxymethyl betaine, cetyl dimethyl betaine and combinations thereof.

For purposes of the present invention, the total of all surfactants to water may be in a weight ratio ranging from about 1:10 to about 5:1, preferably from about 1:4 to about 4:1, more preferably from about 1:3 to about 3:1, and optimally from about 1:2 to about 2:1.

A necessary element of the present invention is that of a water insoluble substrate. By "water insoluble" is meant the substrate does not dissolve or readily break apart upon immersion in water. A wide variety of materials can be used as the substrate. The following non-limiting characteristics are desirable: (i) sufficient wet strength for use, (ii) sufficient abrasivity, (iii) sufficient loft and porosity, (iv) sufficient thickness, and (v) appropriate size.

Non-limiting examples of suitable insoluble substrates which meet the above criteria include non-woven substrates, woven substrates, hydro-entangled substrates, air entangled substrates and the like. Preferred embodiments employ non-woven substrates since they are economical and readily available in a variety of materials. By non-woven is meant that the layer is comprised of fibers which are not woven into a fabric but rather are formed into a sheet, particularly a tissue. The fibers can either be random (i.e., randomly aligned) or they can be carded (i.e. combed to be oriented in primarily one direction). Furthermore, the non-woven substrate can be composed of a combination of layers of random and carded fibers.

Non-woven substrates may be comprised of a variety of materials both natural and synthetic. By natural is meant that the materials are derived from plants, animals, insects or byproducts. By synthetic is meant that the materials are obtained primarily from various man-made materials or from material that is usually a fibrous web comprising any of the common synthetic or natural textile-length fibers, or mixtures thereof.

Non-limiting examples of natural materials useful in the present invention are silk fibers, keratin fibers and cellulosic fibers. Non-limiting examples of keratin fibers include those selected from the group consisting of wool fibers, camel hair fibers, and the like. Non-limiting examples of cellulosic fibers include those selected from the group consisting of wood pulp fibers, cotton fibers, hemp fibers, jute fibers, flax fibers, and mixtures thereof.

Non-limiting examples of synthetic materials useful in the present invention include those selected from the group consisting of acetate fibers, acrylic fibers, cellulose ester fibers, modacrylic fibers, polyamide fibers, polyester fibers, polyolefin fibers, polyvinyl alcohol fibers, rayon fibers and mixtures thereof. Examples of some of these synthetic materials include acrylics such as Acrilan®, Creslan®, and the acrylonitrile-based fiber, Orlon®; cellulose ester fibers such as cellulose acetate, Arnel®, and Acele®; polyamides such as Nylons (e.g., Nylon 6, Nylon 66, and Nylon 610); polyesters such as Fortrel®, Kodel®, and Dacron®; polyolefins such as polypropylene, polyethylene; polyvinyl acetate fibers and mixtures thereof.

Non-woven substrates made from natural materials consist of webs or sheets most commonly formed on a fine wire screen from a liquid suspension of the fibers.

Substrates made from natural materials useful in the present invention can be obtained from a wide variety of commercial sources. Non-limiting examples of suitable commercially available paper layers useful herein include Airtex®, an embossed airlaid cellulosic layer having a base weight of about 77.6 gsm (71 gsy), available from James River Corporation, Green Bay, WI; and Walkisoft®, an embossed airlaid cellulosic having a base weight of about 82.0 gsm (75 gsy), available from Walkisoft U.S.A., Mount Holly, NC.

Non-woven substrates made from synthetic material useful in the present invention can also be obtained form a wide variety of commercial sources. Non-limiting examples of suitable non-woven layer materials useful herein include HFE- 40-047, an apertured hydroentangled material containing about 50% rayon and 50% polyester, and having a basis weight of about 47.0 gsm (43 grams per square yard (gsy)), available from Vertec, Inc., Walpole, MA; HEF 140-102, an apertured hydro-entangled material containing about 50% rayon and 50% polyester, and having a basis weight of about 61.2 gsm (56 gsy), available from Veratec, Inc., Walpole, MA; Novenet® 149-191, a thermo-bonded grid patterned material containing about 69% rayon, about 25% polypropylene, and about 6% cotton, and having a basis weight of about 109.4 gsm (100 gsy), available from Veratec, Inc., Walpole, MA; HEF Nubtex® 149-801, a nubbed, apertured hydro-entangled material, containing about 100% polyester, and having a basis weight of about 76.6 gsm (70 gsy), available from Veratec, Inc. Walpole, MA; Keybak® 951V, a dry formed apertured material, containing about 75% rayon, about 25% acrylic fibers, and having a basis weight of about 47.0 gsm (43 gsy), available from Chicopee Corporation, New Brunswick, NJ; Keybak® 1368, an apertured material, containing about 75% rayon, about 5% polyester, and having a basis weight of about 42.7 gsm (39 gsy), available from Chicopee Corporation, New Brunswick, NJ; Duralace® 1236, an apertured, hydro-entangled material, containing about 100% rayon, and having a basis weight from about 43.8 gsm (40 gsy) to about 125.8 gsm (115 gsy), available from Chicopee Corporation, New Brunswick, NJ; Duralace® 5904, an apertured, hydro-entangled material, containing about 100% polyester, and having a basis weight from about 43.7 gsm (40 gsy) to about 125.8 gsm (115 gsy), available from Chicopee Corporation, New Brunswick, NJ; Sontaro® 8868, a hydro-entangled material, containing about 50% cellulose and about 50% polyester, and having a basis weight of about 65.6 gsm (60 gsy), available from Dupont Chemical Corp.

Most preferred as a substrate for purposes of this invention are non-woven substrates, especially blends of rayon/polyester and ratios of 10:90 to 90:10, preferably ratios of 20:80 to 80:20, optimally 40:60 to 60:40 by weight. A most useful substrate is a 70:30 rayon/polyester non-woven wipe article.

Anywhere from 1 to 100, preferably from 5 to 50 single wipes may be stored within a dispensing pouch or container, preferably a moisture impermeable pouch or container.
During storage and between dispensing, the pouch or container is preferably resealable. Single wipe containing pouches may also be employed.

The water insoluble substrates of the present invention can comprise two or more layers, each having a different texture and abrasiveness. The differing textures can result from the use of different combinations of materials or from the use of a substrate having a more abrasive side for exfoliation and a softer, absorbent side for gentle cleansing. In addition, separate layers of the substrate can be manufactured to have different colors, thereby helping the user to further distinguish the surfaces. Although the present invention may be suitable for substrates with two or more layers having different texture and abrasiveness, the best effectiveness of the damp system can be found with single or multiple layered substrates of identical construction.

The amount of impregnating composition relative to the substrate may range from about 20:1 to 1:20, preferably from 10:1 to about 1:10 and optimally from about 2:1 to about 1:2 by weight.

Impregnating compositions of the present invention may also include silicones of a volatile and non-volatile variety. Typical volatile silicones are the cyclomethicones commercially available as Dow Corning 244, 245, 344 and 345. Linear volatile dimethicones are also suitable. Non-volatile silicones include polydimethyl siloxanes of a viscosity greater than 2 centistoke and silicone copolyols also known as dimethicone copolyol for which Dow Corning 193 is a commercial source. Amounts of the silicones may range from about 0.01 to about 20, preferably from about 0.5 to about 3% by weight of the impregnated composition.

Cationic conditioning agents in monomeric and polymeric type are also useful for purposes of this invention. Examples of the polymeric type include: cationic cellulose derivatives, cationic starches, copolymers of a diallyl quaternary ammonium salt and an acrylamide, quaternized vinylpyrrolidone vinylimidazole polymers polyglycol amine condensates, quaternized collagen polypeptide, polyethylene imine, cationized silicon polymer (e.g. Amodimethicone), cationic silicon polymers provided in a mixture with other components under the trademark Dow Corning 929 (cationized emulsion), copolymers of adipic acid and dimethylaminohydroxypropyl diethylenetriamine, cationic chitin derivatives, cationized guar gum (e.g. Jaguar C-B-S, Jaguar C-17, Jaguar C-16 etc. manufactured by the Celanese Company), quaternary ammonium salt polymers (e.g. Mirapol A-15, Mirapol AD-1, Mirapol AZ-1, etc., manufactured by the Miranol Division of the Rhone Poulenc Company). Most preferred is polyquaternium-11 available as Luviquat® PQ 11 sold by the BASF Corporation.

Examples of monomeric cationic conditioning agents are salts of the general structure: 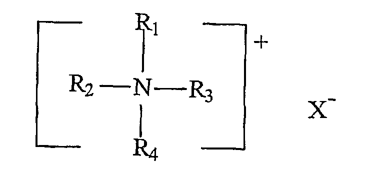 wherein R¹ is selected from an alkyl group having from 12 to 22 carbon atoms, or aromatic, aryl or alkaryl groups having from 12 to 22 carbon atoms; R², R³, and R⁴ are independently selected from hydrogen, an alkyl group having from 1 to 22 carbon atoms, or aromatic, aryl or alkaryl groups having from 12 to 22 carbon atoms; and X⁻ is an anion selected from chloride, bromide, iodide, acetate, phosphate, nitrate, sulfate, methyl sulfate, ethyl sulfate, tosylate, lactylate, citrate, glycolate, and mixtures thereof. Additionally, the alkyl groups can also contain ether linkages, or hydroxy or amino group substituents (e.g. the alkyl groups can contain polyethylene glycol and polypropylene glycol moieties). Preferably the anion is phosphate, especially preferred is hydroxy ethyl cetyl dimonium phosphate available as Luviquat® Mono CP from the BASF Corporation.

Amino silicones quats may similarly be employed. Most preferred is Silquat AD designated by the CTFA as Silicone Quaternium 8, available from Siltech Inc.

Amounts of each cationic agent may range from about 0.05 to about 5%, preferably from about 0.1 to about 3%, optimally from about 0.3 to about 2.5% by weight of the impregnated composition.

The disposable, single use personal care cleansing products of the present invention are manufactured by separately or simultaneously adding onto or impregnating into a water insoluble substrate the cleansing composition including lathering surfactants and conditioners, wherein the resulting product is damp. By "separately" is meant that the surfactants and the conditioners can be added sequentially, in any order without first being combined together. By "simultaneously" is meant that the surfactants and conditioners can be added at the same time, with or without first being combined together.

The surfactant, conditioners, water-binding agents and any other optional ingredients can be added onto or impregnated into the water insoluble substrate by any means known to those skilled in the art. For example, addition can be through spraying, laser printing, splashing, dipping, soaking, or coating.

Except in the operating and comparative examples, or where otherwise explicitly indicated, all numbers in this description indicating amounts of material ought to be understood as modified by the word "about".

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

The following examples will more fully illustrate the embodiments of this invention. All parts, percentages and proportions referred to herein and in the appended claims are by weight unless otherwise illustrated.

### EXAMPLE 1

A formula typical of the present invention impregnated onto a rayon/polyester substrate was formulated with the following components.

**TABLE I**

| Base Formula | |
|---|---|
| **Ingredient** | **Weight %** |
| Deionized Water | 26.16 |
| Cocamidopropyl Betaine (Tegobetaine F®, 30% Active) | 27.00 |
| Sodium Lauroyl Sarcosinate (Hamposyl L-30®, 30% Active) | 27.00 |
| Decyl Polyglucoside (Plantareen 2000 N®, 50% Active) | 18.00 |
| Silicone Quaternium-8 (Hanisquat AD®, 40% Active) | 0.50 |
| Polyquaternium 10 (Celquat SC-230M®) | 0.40 |
| Fragrance | 0.40 |
| Sodium Lauroyl Lactylate (Pationic 138C®) | 0.20 |
| Polyquaternium 4 (Celquat L-200®) | 0.15 |
| Methyl Paraben | 0.15 |
| Soya Sterol (Generol 122®) | 0.01 |
| Cholesterol | 0.01 |
| Vitamin E Acetate | 0.01 |
| Vitamin A Palmitate | 0.01 |

A series of water activities with different water-binding agents are listed for the base formula of Table I. These are recorded in Table II.

**TABLE II**

| **Sample** | **Water Activity** |
|---|---|
| Base Formula (100%) | 0.977 |
| Base Formula (75%) with glycerin (25%) | 0.882 |
| Base Formula (75%) with PEG 9M (25%) | 0.976 |
| Base Formula (94%) with Sodium Chloride (6%) | 0.925 |
| Base Formula (88%) with Dextrose (12%) | 0.964 |
| Base Formula (94%) with Silica (6%) | 0.97 |

The drop in water activity with glycerin is particularly exceptional. Towelettes coated with glycerin performed extremely well in foaming tests as described below.

The base formula (75%) with glycerin (25%) was coated onto a substrate at 2.0 grams coating per 2.0 grams rayon/polyester sheet (152 mm by 190 mm area).

Lather quality/stability comparisons were conducted on the glycerin fortified towelette and two commercial products. A modified Ross-Miles foam tester was employed. The towelettes lather parameters were measured on 3 cloths in 200 ml of water. Results are reported in the Table below.

**TABLE III**

| Lather Quality/Stability Comparison | | |
|---|---|---|
| **Sample** | **Lather** **Quality/Stability*** | **Lather** **Height(M1)** |
| Olay® Daily Facial Cleansing Cloths** | 3 | 400 |
| Dove® Daily Facial Cleansing Cloths | 3 | 550 |
| Base Formula with Glycerin (coated on towelette) | 4.5 | 500 |

| | | |
|---|---|---|
| * Scale of 1 to 5, in order of increasing lather quality/stability. | | |
| ** Representative of towelettes described by P&G in U.S. Patent 5,951,991, U.S. 5,980,931, U.S. 5,952,043, U.S. 5,863,663 and WO 99/55303. | | |

A consumer study was also conducted on two of the towelettes described above. The study was based on response by 118 panelists. Results are reported in Table IV below.

**TABLE IV**

| Consumer Test | | |
|---|---|---|
| | **% Preferred** | |
| **Property** | **Base Formula (75%) /Glycerin (25%) (Coated on Towelette)** | **Olay® Daily Facial Cleansing Cloths** |
| Having a creamy lather | 72 | 28 |
| Lathers quickly | 68 | 32 |
| Leaves skin feeling soft | 61 | 39 |
| Leaves skin feeling moisturized | 59 | 41 |

Based upon the findings reported in Table IV, there was a significant preference for the glycerin/high water towelette.

The Olay® towelette with only 3-4% water and a high water activity constant did not provide a sufficiently creamy lather, lathered slower, left skin feeling less soft and less moisturized than the towelette of the present invention.

### EXAMPLE 2

More than 60 different materials were evaluated as water-binding agents for use in the compositions for impregnation into the towelettes. Most did not work including gums, silicas, calcium carbonate, aluminum hydroxides, aluminum carbonates, mica and calcium chloride. These materials either exhibited a too high water activity or thickened the compositions sufficiently to be unusable with the towelettes. Table VI lists materials which were effective as water-binding agents according to the present invention. Table V is the base formula into which the "water-binding agent" was combined. The base formula was set as a 100% value. For instance, to accommodate 5% of a water-binding agent, 95% of the total composition was base formula. In this manner all ingredients were maintained proportionally equivalent.

**TABLE V**

| **Ingredient** | **Weight %** |
|---|---|
| Deionized Water | 20.60 |
| Water-binding agent | -- |
| Decyl Polyglucoside (Planteren 2000N®, 50% Active) | 18.00 |
| Sodium Lauroyl Sarcosinate (Hamposyl L-30®, 30% Active) | 27.00 |
| Cocoamidopropyl Betaine (Tegobetaine F®, 30% Active) | 27.00 |
| Polyquaternium (Mackernium® 007S) | 5.00 |
| Sodium Lauroyl Lactylate (Pationic 138C®) | 0.30 |
| Silquat AD® | 1.50 |
| Fragrance | 0.40 |
| Preservative | 0.20 |

**TABLE VI**

| **Sample (Weight %)** | **Water Activity** |
|---|---|
| Glycerin (3%) | 0.969 |
| Glycerin (6%) | 0.959 |
| Glycerin (12%) | 0.932 |
| Dextrose (3%) | 0.973 |
| Dextrose (6%) | 0.967 |
| Dextrose (12%) | 0.956 |
| Hexylene Glycol (8%) | 0.963 |
| Sodium Chloride (3%) | 0.948 |
| Sodium Chloride (6%) | 0.918 |
| Sorbitol (3%) | 0.969 |
| Sorbitol (6%) | 0.967 |
| Base Formula | 0.979 |

### EXAMPLE 3

A series of compositions are presented under Table VII reflective of the present invention. These compositions are impregnated onto a non-woven polyester substrate at a 1:1 weight ratio.

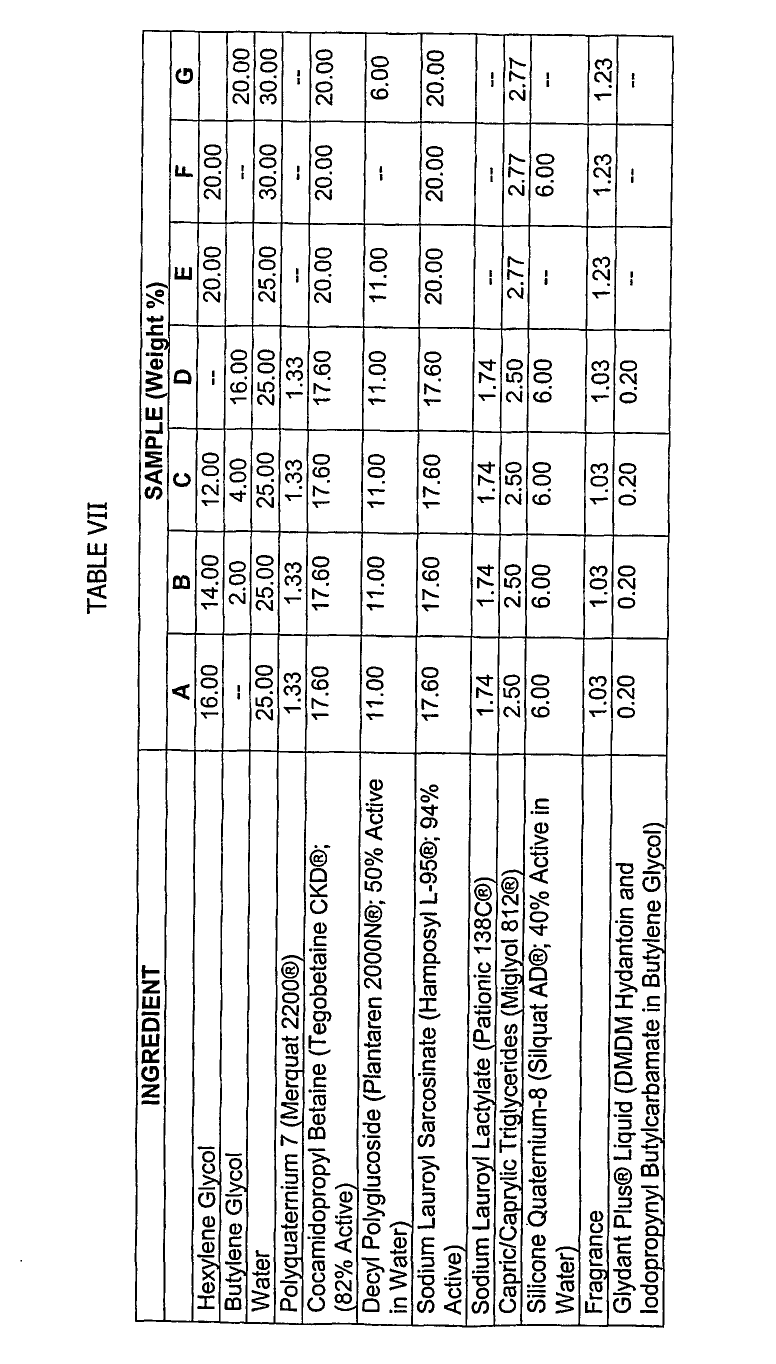

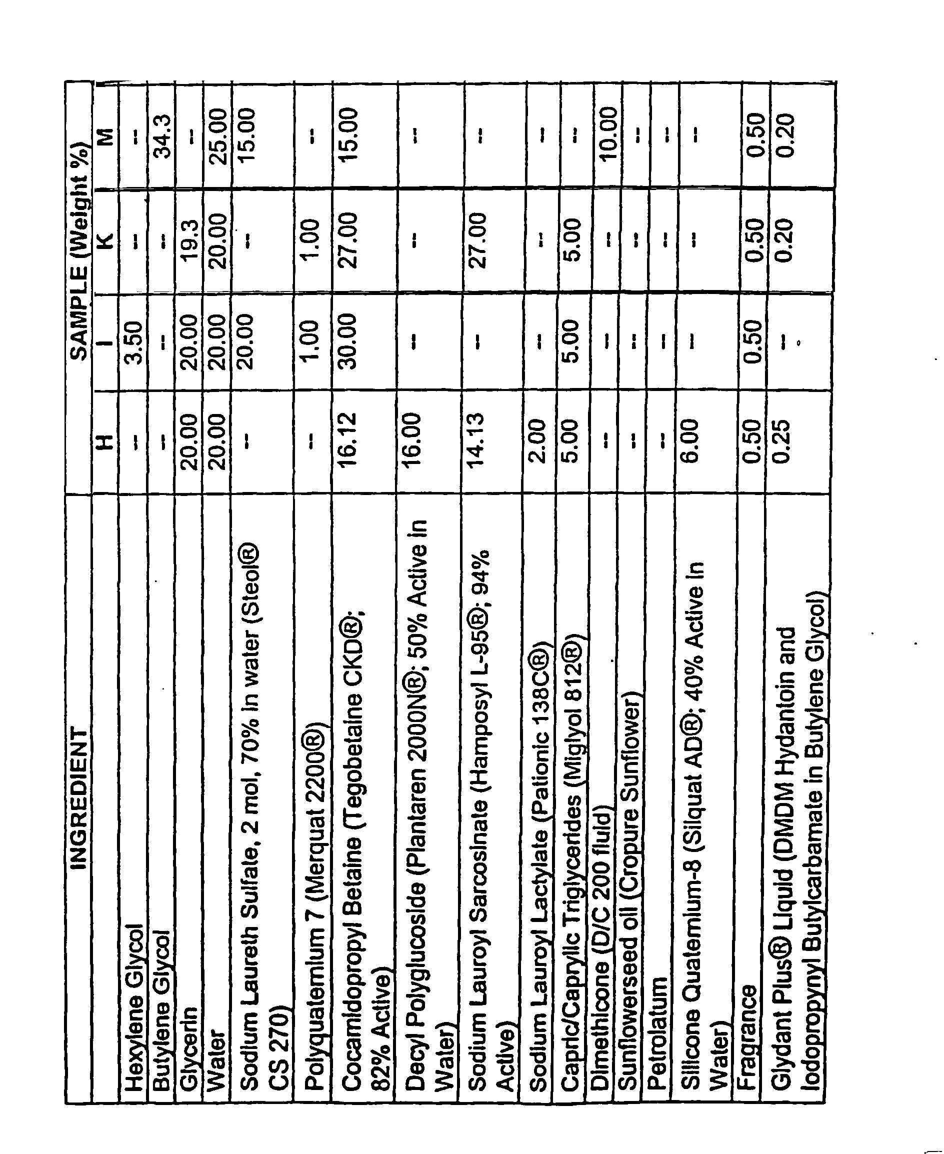

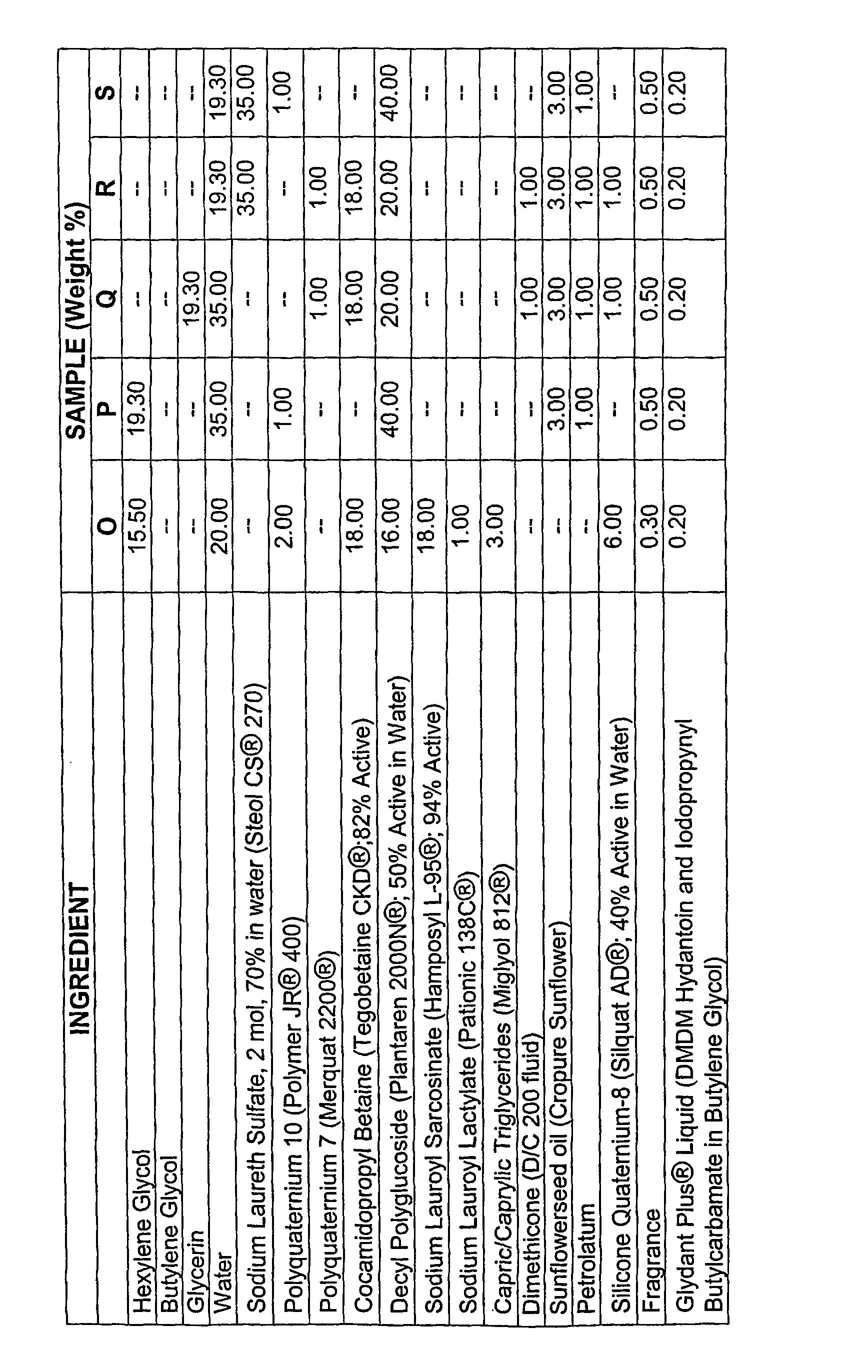

## Claims

1. A damp cleansing product comprising:
(i) a water insoluble substrate; and
(ii) a cleansing composition impregnated onto the substrate comprising:
(a) at least one surfactant present in an amount from 0.5 to 60% by weight of impregnated composition;
(b) water; and
wherein the water is present at greater than 15% to 35% by weight of the product, and from 0.1 to 60% of a water binding agent.

2. The product according to claim 1 wherein the water-binding agent is a polyol or an inorganic salt.

3. The product according to claim 1 or claim 2 wherein the water-binding agent is glycerin.

4. The product according to any of the preceding claims wherein the at least one surfactant is present in an amount from 1 to 20% by weight of total impregnated composition deposited upon the water insoluble substrate.

5. The article according to any of the preceding claims wherein the water insoluble substrate is a sheet selected from non-woven, woven, hydro-entangled and air entangled substrates.

6. The article according to any of the preceding claims further comprising a cationic conditioning agent in amount from 0.05 to 5% by weight of the total composition.

## Patentansprüche

1. Feuchtes Reinigungsprodukt, umfassend:
(i) ein in Wasser unlösliches Substrat und
(ii) eine Reinigungszusammensetzung, die auf das Substrat imprägniert ist, umfassend:
(a) mindestens ein Tensid, das in einer Menge von 0,5 bis 60 Gew.-% der imprägnierten Zusammensetzung vorliegt,
(b) Wasser und
worin das Wasser mit mehr als 15 % bis 35 Gew.-% des Produkts vorliegt und 0,1 bis 60 % eines wasserbindenden Mittels.

2. Produkt nach Anspruch 1, worin das wasserbindende Mittel ein Polyol oder ein anorganisches Salz darstellt.

3. Produkt nach Anspruch 1 oder Anspruch 2, worin das wasserbindende Mittel Glycerin ist.

4. Produkt nach einem der vorangehenden Ansprüche, worin mindestens ein Tensid in einer Menge von 1 bis 20 Gew.-% der gesamten imprägnierten Zusammensetzung, die auf dem in Wasser unlöslichen Substrat abgeschieden ist, vorliegt.

5. Gegenstand nach einem der vorangehenden Ansprüche, wobei das in Wasser unlösliche Substrat ein Tuch ist, ausgewählt aus Vlies, gewebten, wassergelegten und luftgelegten Substraten.

6. Gegenstand nach einem der vorangehenden Ansprüche, weiterhin umfassend ein kationisches konditionierendes Mittel in einer Menge von 0,05 bis 5 Gew.-% der Gesamtzusammensetzung.

## Revendications

1. Produit de nettoyage humide comprenant :
(i) un substrat insoluble dans l'eau ; et
(ii) une composition de nettoyage imprégnée sur le substrat comprenant :
(a) au moins un tensioactif présent en une quantité allant de 0,5 à 60 % en poids de la composition imprégnée ;
(b) de l'eau ; et
dans lequel l'eau est présente à plus de 15 % à 35 % en poids du produit et de 0,1 à 60 % d'un agent se liant à l'eau.

2. Produit selon la revendication 1, dans lequel l'agent se liant à l'eau est un polyol ou un sel inorganique.

3. Produit selon la revendication 1 ou la revendication 2, dans lequel l'agent se liant à l'eau est la glycérine.

4. Produit selon l'une quelconque des revendications précédentes, dans lequel l'au moins un tensioactif est présent en une quantité allant de 1 à 20 % en poids de la composition imprégnée totale déposée sur le substrat insoluble dans l'eau.

5. Article selon l'une quelconque des revendications précédentes, dans lequel le substrat insoluble dans l'eau est une feuille sélectionnée parmi des substrats non tissés, tissés, hyhdro-enchevêtrés par eau et enchevêtrés par air.

6. Article selon l'une quelconque des revendications précédentes, comprenant en outre un agent de conditionnement cationique en une quantité allant de 0,05 à 5 % en poids de la composition totale.
